(19) Europäisches Patentamt / European Patent Office / Office européen des brevets

(11) **EP 3 036 527 B1**

(12) **EUROPEAN PATENT SPECIFICATION**

(45) Date of publication and mention
of the grant of the patent:
**18.04.2018 Bulletin 2018/16**

(51) Int Cl.:
*G01N 21/49* *(2006.01)*      *G01N 33/04* *(2006.01)*
*G01N 21/82* *(2006.01)*      *G01N 21/64* *(2006.01)*

(21) Application number: **14744470.7**

(22) Date of filing: **17.06.2014**

(86) International application number:
**PCT/EP2014/062673**

(87) International publication number:
**WO 2015/000695 (08.01.2015 Gazette 2015/01)**

(54) **A METHOD AND A SYSTEM FOR DETERMINING GEL FIRMNESS VALUES FROM INLINE OPTICAL MEASUREMENTS**

VERFAHREN UND SYSTEM ZUR BESTIMMUNG VON GELFESTIGKEITSWERTEN ANHAND OPTISCHER INLINE-MESSUNGEN

PROCÉDÉ ET SYSTÈME PERMETTANT DE DÉTERMINER DES VALEURS DE FERMETÉ DE GEL À PARTIR DE MESURES OPTIQUES EN LIGNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.07.2013 EP 13382260**

(43) Date of publication of application:
**29.06.2016 Bulletin 2016/26**

(73) Proprietor: **Universitat Autònoma de Barcelona 08193 Cerdanyola del Vallès (ES)**

(72) Inventors:
 • **CASTILLO ZAMBUDIO, Manuel**
  **E-08206 Sabadell (ES)**
 • **ARANGO, Oscar**
  **E-08290 Cerdanyola del Vallès (ES)**

(74) Representative: **Juncosa Miro, Jaime Torner, Juncosa i Associates, S.L. Gran Via de les Corts Catalanes, 669 bis, 1o, 2a 08013 Barcelona (ES)**

(56) References cited:
**US-A1- 2008 268 110      US-A1- 2009 255 473**

 • **MANUEL Z CASTILLO ET AL: "Modelling casein aggregation and curd firming in goats milk from backscatter of infrared light", JOURNAL OF DAIRY RESEARCH, vol. 70, no. 3, 1 August 2003 (2003-08-01) , pages 335-348, XP055149669, ISSN: 0022-0299, DOI: 10.1017/S0022029903006356**
 • **COSTA N E ET AL: "Influence of an exopolysaccharide produced by a starter on milk coagulation and curd syneresis", INTERNATIONAL DAIRY JOURNAL, ELSEVIER APPLIED SCIENCE, BARKING, GB, vol. 22, no. 1, 8 August 2011 (2011-08-08) , pages 48-57, XP028110705, ISSN: 0958-6946, DOI: 10.1016/J.IDAIRYJ.2011.08.004 [retrieved on 2011-09-01]**
 • **O. ARANGO ET AL: "Influence of fat replacement by inulin on rheological properties, kinetics of rennet milk coagulation, and syneresis of milk gels", JOURNAL OF DAIRY SCIENCE, vol. 96, no. 4, 1 April 2013 (2013-04-01), pages 1984-1996, XP055149670, ISSN: 0022-0302, DOI: 10.3168/jds.2012-5763**
 • **CASTILLO M ET AL: "Effect of temperature and inoculum concentration on prediction of both gelation time and cutting time. Cottage cheese-type gels", INTERNATIONAL DAIRY JOURNAL, ELSEVIER APPLIED SCIENCE, BARKING, GB, vol. 16, no. 2, 1 February 2006 (2006-02-01), pages 147-152, XP024963400, ISSN: 0958-6946, DOI: 10.1016/J.IDAIRYJ.2005.02.006 [retrieved on 2006-02-01]**

- CASTILLO M ET AL: "The effect of temperature and inoculum concentration on rheological and light scatter properties of milk coagulated by a combination of bacterial fermentation and chymosin. Cottage cheese-type gels", INTERNATIONAL DAIRY JOURNAL, ELSEVIER APPLIED SCIENCE, BARKING, GB, vol. 16, no. 2, 1 February 2006 (2006-02-01), pages 131-146, XP024963399, ISSN: 0958-6946, DOI: 10.1016/J.IDAIRYJ.2005.02.005 [retrieved on 2006-02-01]

**Description**

Field of the art

The present invention generally relates, in a first aspect, to a method for determining gel firmness values from inline optical measurements, comprising collecting light data and calculating a value of a rheological variable therefrom, and more particularly to a method comprising defining and combining mathematical models of the evolution in time of, respectively, an optical variable and said rheological variable.

A second aspect of the invention concerns to a system adapted to implement the method of the first aspect.

Prior State of the Art

The determining of gel firmness values from inline optical measurements is a long-time wished goal for several kinds of gels which harden and increase their turbidity to develop firmness, in order to monitor and control their evolution in time for several reasons.

Milk gel is one of the most interesting of said gels, because the monitoring and control of its evolution in time can bring many benefits in the manufacturing of different dairy products, particularly cheese, as will be explained below.

Formation of a milk gel and cutting the gel into curd grains to allow whey separation are two major unit operations in cheese making. The gel formation consists of casein micelle colloidal destabilization owing to chemical modification of the protective κ-casein hairy coat by the coagulating agents. The second step consists of aggregation of the destabilized casein micelles to form the gel network. Further cross-linking of casein micelles results in the development of a firm gel. For most cheese types, whey and curd separation does not take place spontaneously and requires cutting the gel into small cubes (curd grains). This operation increases the gel surface/volume ratio, allowing the whey to escape while curd grains contract (Castillo, 2006).

Cutting time (CT) selection depends on rheological and microstructural properties of gels, such as coagulum firmness and rearrangement capability that, in turn, depend on coagulation factors, milk composition, and milk pre-treatment. For this reason, CT selection greatly affects moisture, yield, and quality of cheese and whey fat losses. Cutting and stirring speeds can also exert a marked impact on curd particle size and/or fat losses to the whey at draining. At constant cutting and stirring speeds, cutting the gel too soon enhances the mechanical impact of cutting and stirring operations on curd grains, which increases curd fines and whey fat losses, decreasing cheese yield. By contrast, delaying CT tends to produce the opposite effect on cheese yield. But, excessive delay in cutting also produces an overly firm gel, unable to collapse, which increases curd moisture content. Excessive moisture content causes a "fake" increase in yield and could alter the ripening process, compromising cheese quality (Castillo, 2006; Johnston et al., 1998).

In practice, gel is usually cut after a predetermined reaction time or upon the operator's judgment based on subjective evaluation of textural and visual gel properties. Cutting the curd after a predetermined time is a very common practice, but it is questionable because the factors that affect curd firmness and gel microstructure could vary the optimum CT. Cutting the curd by relying on empirical inspection is accurate and acceptable if the evaluation is made properly. The lack of a rigorous CT characterization and the usual changes in milk protein content are forcing modern cheese plants to standardize the protein content of milk in an attempt to control coagulation, gel firming, curd syneresis, cheese yield, and product quality.

Due to the importance of the milk coagulation stage on the final cheese quality, numerous devices have been developed to study and control the phenomena occurring during rennet-induced coagulation on plant and/or laboratory scale. O'Callaghan et al. (2002) and Castillo (2006) reviewed the various systems developed to monitor coagulum formation and Klandar et al. (2007) compared several methods for assessment of the rennet coagulation of skim milk. On plant scale, an ideal monitoring system should evaluate curd firmness, as distinct from gelation, operate inline in the process vat, be non-intrusive, non-destructive and compatible with hygiene requirements. Concurrent to the traditional practice of cheesemakers, several systems were proposed to assess milk coagulation based on a wide range of mechanical, vibrational, ultrasonic, thermal or optical instrument methods. The Formagraph, based on the drag force technique, was the first mechanical off-line instrument widely used (McMahon & Brown, 1982). It was able to assess rheological changes during milk coagulation but its intrusive behaviour was a disadvantage. Rheological devices such as the low-amplitude dynamic oscillatory rheometer, provide very useful information on the gel formation process (Lucey, 2002). This off-line method, considered as a reference one, was often used to monitor rennet-induced coagulation (Dejmek, 1987; Hemar et al., 2004; Mellema et al., 2002; Zhong & Daubert, 2004). O'Callaghan, et al., (2000) successfully assessed rennet induced coagulation using a tuning-fork and torsional probes. A real potential for coagulation monitoring was attributed to ultrasonic sensing devices (Benguigui et al., 1994; Cosgrove, 2000; Dalgleish et al., 2005; Taifi et al, 2006), but important progress is still lacking in proposing industrial equipment. The hot wire probe, a thermal on-line method, was particularly efficient at predicting gel time, but was ill-adapted to follow firmness evolution (Hori, 1985; O'Callaghan et al., 2001; Passos et al., 1999).

Changes of the optical properties of milk during coagulation have permitted the development of several devices, more particularly since fibre-optics became widespread. Optical techniques such as light absorbance (McMahon et al., 1984), transmission (Najera et al., 2003; O'Callaghan et al., 1999) and reflectance (Castillo et al., 2000; Castillo et al., 2002, Laporte et al., 1998; O'Callaghan et al., 2000; Payne, et al., 1990), over visible and near-infrared (NIR) wavelengths, were employed to assess milk coagulation. Many of those optical methods are very suitable for inline measurement using optical fibres. An optical microscopic method was proposed by Lagaude et al., (2004) for determination of rennet visual flocculation and rheometric gel times and to observe simultaneously structural changes occurring during gel formation. Reflection photometry (colorimeters) has been applied to follow milk coagulation. The coordinates L and b (lightness and blue-yellow, respectively) rise during coagulation, which has been attributed to the increase in the average dimension of casein particles during their aggregation (Hardy and Fanni, 1981). Study of network formation has also been often realized using light scattering. In milk and, especially, in the visible and near infrared ranges, light scattering predominates over absorption. Light scattering is directly related to the rate of aggregation and curd firming if total casein concentration does not vary during the measurements. Indeed, use of optical fibres allows applying different optical configurations for sensor development. Conventional turbidity measurements were one of the first light scattering techniques proposed for monitoring network growth (McMahon et al., 1984). A nondestructive optical device, the Gelograph-NT, which is based on conventional turbidity measurements (transmission), has been developed from the previous rheological method, Gelograph-M (based on an oscillating sensing needle). A 10-channel lab version of the Gelograph-NT, the Optigraph, (Picque et al., 1996) which allows estimating clotting time and CT has been developed.

Direct light backscatter measurements, such as those performed at 880 nm by the inline sensor CoAguLite developed by Payne et al. (1990), have the advantage that the measurements do not depend on a certain path length as compared to indirect light scatter measurements based in relative transmission (Optigraph).

The CoAguLite fibre optic sensor technology has been demonstrated to be one of the most promising inline, non-destructive methods for monitoring milk coagulation. This technique has become commercially available for inline monitoring of cheese production.

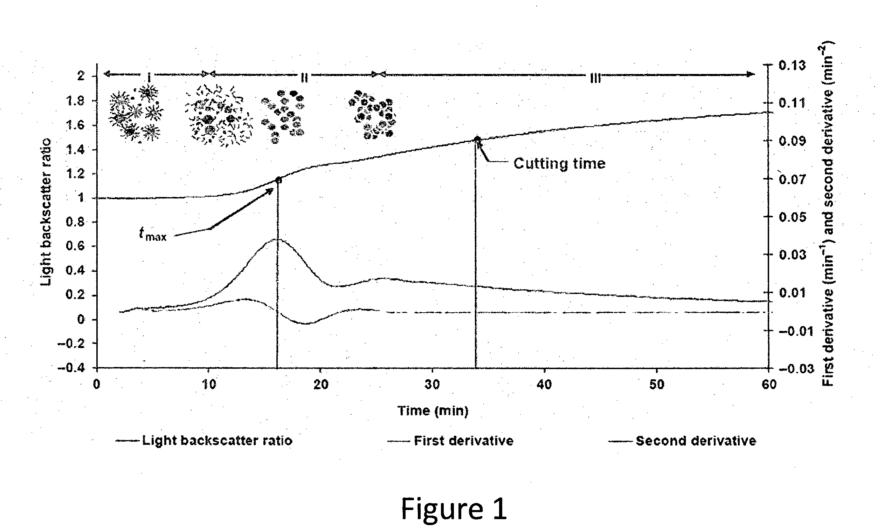

The use of two optical fibres spaced 0.7 mm apart to transport the light is a unique optical configuration that yields a strong signal proportional to the changes that occur in the protein structure during coagulation. Light from a light-emitting diode (LED) is transferred to the milk through a fibre, and the light backscattered from the milk is transmitted through an adjacent fibre to an optical detector. The LB signal contains information about aggregation of casein micelles and gel assembly during milk coagulation. The LB profile increases sigmoidally as milk coagulation proceeds for acid-, mixed-, or rennet-induced coagulation in cow, goat, and sheep milk (raw and skim) (Payne and Castillo, 2007; Nicolau et al., 2010). The LB ratio is generated by dividing the voltage from the sensor, V, by the voltage $V_0$ obtained by averaging over the period of 1 min after adding the enzyme. The LB ratio begins with a value of 1 and represents the increase in signal during coagulation. The LB ratio profile has a latent period (Fig. 1 I) during which enzymatic reactions predominate with no detectable change in backscatter intensity (Payne and Castillo, 2007). The LB ratio increases as particle size increases during network formation due to casein micelle cross-linking. As a result, a sigmoidal period can be distinguished (Fig. 1 II) during which aggregation reactions predominate, as well as an asymptotic period (Fig. 1 III) during which cross-linking proceeds at an ever-decreasing rate while curd firming is developing.

As a consequence of the sigmoidal shape of the LB ratio profile, a unique parameter is obtained (Payne and Castillo, 2007): the time from enzyme addition to the inflection point of the LB ratio profile, $t_{max}$ (Fig. 1). One important discovery was the strong correlation between $t_{max}$ and the enzymatic reaction rate, k (Saputra et al., 1992). Thus, the LB measurement indirectly measures the enzymatic reaction rate when it measures $t_{max}$. Another important discovery was that the onset of aggregation is located between the induction period (the time from enzyme addition to a point at which the magnitude of the first derivative of the normalized LB ratio is equal to 0.025) and $t_{max}$, and that the extent of hydrolysis at $t_{max}$ is near completion (Castillo et al., 2003). Thus, it is not surprising that when temperature, pH, added calcium, or enzyme concentration change, $t_{max}$ changes accordingly.

The backscatter parameter, $t_{max}$, is highly correlated with cutting time, as objectively determined using the Formagraph parameter $k_{20}$. The following prediction equation was developed by Payne et al. (1993) to predict cutting time ($t_{cut}$), $t_{cut} = \beta t_{max}$. The regression coefficient $\beta$ typically varies between 1.15 and 2.4, depending on the enzyme used and the type of milk and product produced. The coefficient $\beta$ is selected in the plant to replicate the cheese maker's judgment of cutting time. The value of $\beta$ calibrates the system and is the only variable that the cheese maker has to determine. The equation for $t_{max}$ is accurate only if protein is constant. Castillo (2001) showed that $\beta$ decreases significantly when protein increases. This allowed the development of a prediction algorithm to correct the cutting time prediction for changes in milk protein content as follows: $t_{cut} = \beta_0 t_{max}(1+\gamma P)$, where the constant $\gamma$ corrects the cutting time prediction for the effect of protein concentration (P). Thus, the regression coefficient $\beta_0$ is the only parameter that requires in-plant calibration.

Rennet-induced gels are viscoelastic and their rheological properties can be characterized using dynamic low-amplitude oscillatory rheology, which determines both the viscous and elastic moduli (Lucey, 2002). Parameters that can be determined include the elastic or storage modulus (G'), which is a measure of the energy stored per oscillation cycle, the viscous or loss modulus (G"), which is a measure of the energy dissipated as heat per cycle, the complex modulus (G*), G*= [(G')$^2$ + (G")$^2$]$^{1/2}$ and loss tangent (tan$\delta$), which is the ratio of the viscous to elastic properties. These parameters are defined as follows:

$$G' = \left(\frac{\tau_0}{\gamma_0}\right) cos\delta$$

$$G'' = \left(\frac{\tau_0}{\gamma_0}\right) sin\delta$$

$$tan\delta = \frac{G''}{G'}$$

Where $\tau_0$ is the amplitude of the shear stress, $\gamma_0$ is the amplitude of the strain, and $\delta$ is the phase angle. The parameter tan$\delta$ is related to the relaxation of bonds in the gel during deformation and is a useful parameter for characterizing milk gels.

During gelation, there is a lag period before a measurable G' value is obtained. The value of tan$\delta$ decreases at the gelation point and then attains a relatively constant value. The dynamic moduli increase relatively rapidly initially and then, after a period of several hours, tend to plateau. The increase in the moduli after gelation probably reflects ongoing fusion of micelles, which results in an increase in the contact area between aggregated particles, and possibly the incorporation of additional particles into the gel network. Typical plateau values for the storage modulus of rennet induced gels range from 100 to 200 Pa. Both moduli have lower values at low frequencies, reflecting relaxation of more bonds when the time-scale of the applied stress is longer. There is also an increase in tan$\delta$ with decreasing frequency. Changes in tan$\delta$, measured at constant frequency, have been associated with an increased susceptibility of renneted milk gels to undergo syneresis.

The main reason for modelling the coagulation process is to have an objective way to characterize gelation process and the effect of treatments, such as type of coagulant and milk composition, among many others, on the various model parameters. Another objective is to try to standardize the gel firmness at cutting as this is thought to be important for controlling yield and cheese moisture content. There is a lack of an efficient and reliable, non-destructive, easy to clean and sanitary, inline monitoring curd firming sensor, which is seriously hindering complete cheesemaking automation for more than six decades.

Many attempts have been made to produce mathematical equations to predict the growth of gel firmness with time. Models range from the purely empirical approach (Scott Blair and Burnett, 1958) to those rooted in some kinetic mechanism (Tuszynski, 1971; Douillard, 1973; Carlson et al., 1987; Clark and Amici, 2003). Tested simply on their ability to fit the observed growth curves, some are more successful than others, which fail to reproduce salient features (Fox et al., 2004). Others have no theoretical basis which makes them less useful as predictive tools. Scott Blair and Burnett (1958) proposed an empirical model as follows:

$$G(t) = \mathrm{G}_\infty \exp\left(\frac{-\tau}{t - t_\mathrm{g}}\right)$$

to describe the increase in shear modulus (G) with time beyond the gel point occurring at gelation time, $t_\mathrm{g}$, $\tau$ being a constant characteristic of the sample and determined by fitting. Dejmek (1987) demonstrated that this model provides a good fit to experimentally obtained cure curves but has non-random residuals, indicating that the function may not be appropriate for its intended purpose.

Douillard (1973) proposed a model in which the rate of change of shear modulus with time followed first-order kinetics:

$$\frac{dG}{dt^*} = k(G_\infty - G)\, t^* \geq 0 \; or \; t \geq t_g$$

This equation can be integrated to give:

$$G = G_\infty[1 - \exp(-kt^*)]$$

with the initial condition that G = 0 at $t = t_g$, where $t^* = t - t_g$, $G_\infty$ is the value of the shear modulus at $t = \infty$ and $k$ is the rate constant for the process but essentially a fitting parameter.

This equation has recurred several times in the history of studies on the rennet coagulation of milk. Tokita et al. (1982) fitted their cure curves to an $n^{th}$-order reaction equation and determined that the first-order form, the Douillard equation above, gave the best fit. They further demonstrated that the gel time, $t_g$, obtained in these studies varied inversely with enzyme concentration, and that the rate parameter, $k$, had a power-law dependence on enzyme concentration with an exponent of 0.8.

Another optical technique which has been disclosed (Fagan et al., 2011) as susceptible to be used as an inline, non-destructive method for monitoring milk coagulation, is fluorescence spectroscopy, where light is emitted into milk, generally via an optical fibre. Endogen fluorophores in the sample radiate light almost instantaneously upon being struck by the emitting light and the generated fluorescence light is received in a detector, generally via another optical fibre, and analysed over time to evaluate milk coagulation and curd syneresis, where the evolution over time of the fluorescence profile of some of the mentioned endogen fluorophores (specifically tryptophan and riboflavin) also increases sigmoidally during coagulation.

In U.S. Patent N° 6,831,741 B1 (Dec, 14, 2004) an arrangement and method for determining gel firmness values from inline optical measurements is disclosed, consisting in applying diffusing wave spectroscopy to measure the properties of multiphase systems, as well as the changes in the dynamic light scattering apparatus. Such a method may be related to monitoring the renneting of cheesemilk during cheesemaking and obtain the gel-strength (G') using the following equation:

$$G' \approx \frac{k_b T}{\xi(\Delta r_m^2)}$$

In which,

$k_b T$ = thermal energy of particles in the gel;
$\xi$ = size of a cluster in the gel;
$\Delta r_m^2$ = maximum value of the mean square displacement.

The method for G' calculation in US 6,831,741 B1 use dynamic light scattering, which is a complex and expensive system. On the other hand, the method for calculation of gel-strength in US 6,831,741 B1 is based on fractal theory so it requires knowledge of a number of theoretical parameters that must be measured or known previously.

No other method is known by the present inventors for determining gel firmness values from inline optical measurements, neither from light scattering measurements nor from fluorescence light measurements nor from any other kind of optical measurements.

REFERENCES:

Arango, O., Trujillo, A. J., Castillo, M. (2013). Influence of fat replacement by inulin on rheological properties, kinetics of rennet milk coagulation, and syneresis of milk gels. Journal of Dairy. 96, 1984-1996.

Benguigui, L., Emery, J., Durand, D., & Busnel, J. P. (1994). Ultrasonic study of milk-clotting. Lait, 74, 197-206.

Cosgrove, N. (2000). Design and development of an ultrasonic measuring system to monitor milk coagulation. Thesis presented to National University of Ireland for award of MEngnSc, Dublin, Ireland.

Carlson, A., Hill Jr., C.G. and Olson, N.E (1987). Kinetics of milk coagulation. III. Mathematical modelling of the kinetics of curd formation following enzymatic hydrolysis of K-casein- parameter estimation. Biotechnol. Bioeng. 29, 601-611.

Castillo, M. (2006). Cutting time prediction methods in cheese making. In: Encyclopedia of Agricultural, Food, and Biological Engineering. Volume 1, Edition 1, Pp. 1-7. Heldman, D., Ed. Taylor & Francis Group, Boca Raton. ISBN:

978-0-8247-0938-9 (hardback); 978-0-8247-0937-2 (electronic).

Castillo, M.; Payne, F.A.; Hicks, C.L.; Laencina, J.S.; Lopez, M.B. (2003). Modeling casein aggregation and curd firming in goats' milk from backscatter of infrared light. J. Dairy Res. 70, 335-348.

Castillo, M., Payne, F. A., Hicks, C. L., Laencina, J., & Lopez, M. B. (2002). Effect of calcium and enzyme in cutting time prediction of coagulating goats' milk using a light scattering sensor. International Dairy Journal, 12, 1019-1023.

Castillo, M., Payne, F. A., Hicks, C. L., & Lopez, M. B. (2000). Predicting cutting and clotting time of coagulating goats' milk using diffuse reflectance: Effect of pH, temperature and enzyme concentration. International Dairy Journal, 10, 551-562.

Castillo, M. (2001). Cutting time prediction during cheese making by near infrared light backscattering, PhD thesis, University of Murcia, Spain.

Clark, A.H. and Amici, E.H. (2003). The formation and properties of biopolymer gels, in, Food Colloids and Materials, Dickinson, E. and Van Vliet, T. eds, Royal Society of Chemistry, Cambridge. pp. 35-48.

Dalgleish, D. G., Verespej, E., Alexander, M., & Corredig, M. (2005). The ultrasonic properties of skim milk related to the release of calcium from casein micelles during acidification. International Dairy Journal, 15, 1105-1112.

Dejmek, P. (1987). Dynamic rheology of rennet curd. J. Dairy Sci. 70, 1325-1330.

Douillard, R. (1973). Rheological analysis of curd formation. J. Texture Studies 4, 158-165.

Fox, P. F.; McSweeney, P. L.; Cogan, T. M.; Guinee, T. P. (2004). Cheese: Chemistry, Physics and Microbiology (3rd Edition). Edited by P.F. Fox, An Aspen Publication. 404 p.

Hardy, J.; Fanni, J. (1981). Application of reflection photometry to the measurement of milk coagulation. J. Food Sci. 46, 1956-1957.

Hemar, Y., Singh, H., & Home, D. S. (2004). Determination of early stages of rennet-induced aggregation of casein micelles by diffusing wave spectroscopy and rheological measurements. Current Applied Physics, 4, 362-365.

Hori, T. (1985). Objective measurements of the process of curd formation during rennet treatment of milks by the hot-wire method. Journal of Food Science, 50, 911-917.

Johnston, K.A.; Luckman, M.S.; Lilley, H.G.; Smale, B.M. (1998). Effect of various cutting and stirring conditions on curd particle size and losses of fat to the whey during cheddar cheese manufacture in Ost vats. Int. Dairy J. 8, 281-288.

Klandar, A.H.; Lagaude, A.; Chevalier-Lucia D. (2007). Assessment of the rennet coagulation of skim milk: A comparison of methods. International Dairy Journal 17, 1151-1160.

Lagaude A., Fernandez L., Cuq J.-L., Marchesseau S. (2004) Characterization of curd formation during the rennet coagulation of milk by an optical microscopic method. International Dairy Journal. 14, 1033-1039.

Laporte, M.-F., Martel, R., & Paquin, P. (1998). The near-infrared optic probe for monitoring rennet coagulation in cows' milk. International Dairy Journal, 8, 659-666.

Lucey, J. A. (2002). Formation and physical properties of milk protein gels. Journal of Dairy Science, 85, 281-294.

McMahon, D. J., & Brown, R. J. (1982). Evaluation of formagraph for comparing rennet solutions. Journal of Dairy Science, 65, 1639-1642.

McMahon, D.J.; Brown, R.J.; Richardson, G.H.; Ernstrom, C.A. (1984). Effects of calcium, phosphate, and bulk culture media on milk coagulation properties. J. Dairy Sci. 67, 930-938.

Mellema, M., Walstra, P., Van Opheusden, J. H. J., & Van Vliet, T. (2002). Effects of structural rearrangements on the rheology of rennet-induced casein particle gels. Advances in Colloid and Interface Science, 98, 25-50.

Najera, A. I., de Renobales, M., & Barron, L. J. R. (2003). Effects of pH, temperature, CaCl2 and enzyme concentrations on the rennet-clotting properties of milk: A multifactorial study. Food Chemistry, 80, 345-352.

Nicolau, N., Castillo, M., Buffa, M, O'Callaghan, D. J., Guamis, B. (2010). Estudio de la coagulacion de leche de oveja mediante monitorización con un sensor óptico. Actas del VI Congreso Español de Ingeniería de Alimentos, Logroño, España. ISBN: 978-84-7359-654-1.

O'Callaghan, D. J., O'Donnell, C. P., & Payne, F. A. (2002). Review of systems for monitoring curd setting during cheesemaking. International Journal of Dairy Technology, 55, 65-74.

O'Callaghan, D. J., Mulholland, E. P., Duffy, A. P., O'Donnell, C. P., & Payne, F. A. (2001). Evaluation of hot wire and optical sensors for online monitoring of curd firmness during milk coagulation. Irish Journal of Agricultural and Food Research, 40,227-238.

O'Callaghan, D. J., O'Donnell, C. P., & Payne, F. A. (2000). On-line sensing techniques for coagulum setting in renneted milks. Journal of Food Engineering, 43, 155-165.

O'Callaghan, D. J., O'Donnell, C. P., & Payne, F. A. (1999). A comparison of on-line techniques for determination of curd setting time using cheese milks under different rates of coagulation. Journal of Food Engineering, 41, 43-54.

Passos, E. F., Monteiro, P. S., Oliveira, R. C., Martins, J. G. O., Alves, H. G., & Brandao, S. C. C. (1999). Predicting the cutting time of coagulating milk for cheese production using a heated thermistor. Journal of Food Science, 64, 879-882.

Payne, F. A., Castillo M. 2007. Light backscatter sensor applications in milk coagulation. In: Encyclopedia of Agricultural, Food, and Biological Engineering. Volume 1, Edition 1, Pp. 1-5. Heldman, D., Ed. Taylor & FrancisGroup, Boca Raton. ISBN: 978-0-8247-0938-9 (hardback); 978-0-8247-0937-2 (electronic).

Payne, F.A.; Hicks, C.L.; Shen, P.S. (1993). Predicting optimal cutting time of coagulating milk using diffuse reflectance. J. Dairy Sci. 7648-7661.

Payne, F.A.; Madangopal, S.; Hicks, C.L.; Shearer, S.A. (1990). Fiber optic milk coagulation sensor for cut-time detection. Food Processing Automation Conference, American Society of Agricultural Engineers, St. Joseph. Michigan, Publication 02-90, 173.

Picque, D.; Cattenoz, T.; Latrille, E.; Perret, B.; Corrieu, G. (1996). Dispositif et procede de controle de la fabrication de fromages. INRA patent no 96 12022.

Saputra, D.; Payne, F.A.; Lodder, R.A.; Shearer, S.A. (1992). Selection of near-infrared wavelengths for monitoring milk coagulation using principal component analysis. Trans. ASAE,35, 1597-1605.

Scott Blair, G.W. and Burnett, J. (1958). Physical changes in milk caused by the action of rennet..1. Dairy Res. 25, 297-303.

Tabayehnejad, N., Castillo, M., Payne, F. A. 2011. Comparison of total milk-clotting activity measurement precision using the Berridge clotting time method and a proposed optical method. Journal of Food Engineering, 108, 549-556.

Taifi, N., Bakkali, F., Faiz, B., Moudden, A., Maze, G., & Decultot, D. (2006). Characterization of the syneresis and the firmness of the milk gel using an ultrasonic technique. Measurement Science and Technology, 17, 281-287.

Tokita, M.K., Kikichi, R., Niki, R. and Arima, S. (1982). Dynamic viscoelastic studies on the mechanism of milk clotting process. Biorheology 19,209-219.

Tuszynski, W.B. (1971). A kinetic model of the clotting of casein by rennet. J. Dairy Res. 38, 113-125.

Zhong, Q., & Daubert, C. R. (2004). Kinetics of rennet casein gelation at different cooling rates. Journal of Colloid and Interface Science, 279, 88-94.

The article "Modelling casein aggregation and curd firming in goats milk from backscatter of infrared light", Journal of Dairy Research, Volume 70, Number 3, pages 335-348, 2003-08-01, Manuel Z Castillo et al discloses a method of modelling casein aggregation and curd firming in goats' milk from backscatter of infrared light. The kinetic model describes the curd assembly of skimmed goats' milk during enzymic coagulation which is monitored using an optical sensor that measures diffuse reflectance (light backscatter) at 880 nm. The appearance of a shoulder, at low temperatures and protein concentrations, in the diffuse reflectance ratio profile after the inflection point of the curve (Tmax) appears to separate the aggregation and curd firming steps. The diffuse reflectance ratio profile after Tmax was attributed to the overlapping of casein micelles aggregation and curd firming reactions. The developed kinetic model combined a second order reaction model to describe aggregation reactions and a first order reaction model to describe firming processes reactions.

Description of the Invention

It is necessary to offer an alternative to the state of the art which covers the gaps found therein, by the provision of a method and a system less complicated than those of US 6,831,741 B1, which requires of equipment simpler and less expensive and which can work, for the practical applications, based only on empirical parameters, obtained by calibration in real conditions.

To that end, the present invention provides a method for determining gel firmness values from inline optical measurements as defined in appended independent claim 1. Contrary to the method disclosed in US 6,831,741 B1, the method of the first aspect of the present invention comprises:

- defining a mathematical model, valid for the kinetics of the gelation of said gel, of the evolution in time of said rheological variable;
- defining a mathematical model, valid for the kinetics of the gelation of said gel, of the evolution in time of an optical variable generated from a light generation mechanism responsible for the generation of said detected light;
- combining both mathematical models in order to obtain an expression which does not include time and which relates said rheological variable with said optical variable; and
- introducing said collected light data into said expression to calculate said at least one value of the rheological variable as well as further steps defined in appended independent claim 1. For an embodiment, said gel is increasing its turbidity during its hardening, said light generation mechanism is light scattering, said detected light being said emitted light once scattered by said gel and said collected light data being collected scattered light data, and the method comprises, contrary to the method disclosed in US 6,831,741 B1, using static light scattering for performing said optical detection and collection of scattered light data.

According to an alternative embodiment, said gel is increasing its endogenous fluorescence during its hardening, said light generation mechanism is light fluorescence performed by endogen fluorophores (preferably tryptophan and riboflavin) of said gel upon said emitted light impinges thereon, said detected light being fluorescent light generated by said endogen fluorophores and said collected light data being collected fluorescent light data.

For an embodiment, the method of the first aspect of the invention comprises performing said collecting of light data a plurality of times through the hardening and turbidity or endogenous fluorescence increasing of the gel, and calculating the values of the rheological variable for each of said plurality of times.

Depending on the embodiment, said gel is a casein gel, such as a a milk gel, such as curd (generally for making cheese), yogurt, fermented/acidified milk or milk derived product, although for other less preferred embodiments the gel is of another type, such as starch gels or soya "milk" gels, the invention being valid for any gel that can develop firmness and that suffers any optical change which allows generating at least an optical parameter that would follow a first order kinetics. The rheological variable is the storage modulus G'. In preferred embodiments combination of G' with either one of the viscous modulus G", the phase angle $\delta$, the tangent of said $\delta$ value, tan$\delta$, are also used. Preferably said light scattering of both, said optical variable and said optical detection, is light backscattering, although the method is also valid for other kind of light scatterings, such as light side scattering, or a combination thereof. The mathematical model of said optical variable follows the next equation:

$$R - Ro = (R_\infty - R_0)(1 - e^{-k_R t}) \qquad (1)$$

where,

$R - Ro$ = the increase in the light scatter or endogenous fluorescence ratio, after $t_{2min}$, resulting exclusively from gel firming reactions, where $t_{2min}$ is the time at which there is a minimum in the second derivative of a sigmoidal shaped portion of the light scattering or endogenous fluorescence ratio profile corresponding to said evolution in time of said optical variable;

$R_\infty$ = asymptotic value at infinite time for the light scattering or endogenous fluorescence ratio;

$k_R$ = a first order rate constant for gel firming reaction;

$t$ = time after $t_{2min}$;

said mathematical model of said rheological variable follows the next equation:

$$G' = G'_\infty + (G'_0 - G'_\infty)e^{-k_{G'}t} \qquad (2)$$

where,

$G'$ = the storage modulus at t time;

$G'_\infty$ = the value of the storage modulus at t = ∞;

$k_G$ = the rate constant for the process;

and the method comprises solving for time in equation (1) and replacing it into the equation (2), to obtain, as said expression which does not include time and which relates said rheological variable with said optical variable, the next expression:

$$G' = G'_\infty + (G'_0 - G'_\infty)\left(\frac{R-R_\infty}{R_0-R_\infty}\right)^{k_{GR}} \qquad (3)$$

where $k_{GR} = k_G/k_R$ is a constant representing the ratio between the rates of increase of $G'$ and $R$ values as a result of gel firming.

Equation (1) is based on the teachings of Castillo et al. (2003), which proposed that the curd assembly consists of two different reactions occurring simultaneously. The first reaction is the aggregation of casein micelles and was assumed to follow second order kinetics. The second reaction is curd firming and was assumed to follow first order kinetics. Assuming first-order behaviour for disappearance of crosslinking sites and a linear correlation between light scatter or endogenous fluorescence and the disappearance of crosslinking sites, the curd firming process can be described by said equation (1).

Equation (2)is adapted from the Douillard model for the rate of change of storage modulus with time as follows:

$$G' = G'_0 + (G'_\infty - G'_0)(1 - e^{-k_{G'}t})$$

$$G' = G'_\infty + G'_0 e^{-k_{G'}t} - G'_\infty e^{-k_{G'}t}$$

$$G' = G'_\infty + (G'_0 - G'_\infty)e^{-k_{G'}t},$$

i.e. the indicated above as Equation (2)

The above mentioned solving for time in equation (1) and replacing it into the equation (2), to obtain expression (3) includes the next intermediate steps:

$$R = R_\infty + (R_0 - R_\infty)e^{-k_R t}$$

$$t = \frac{-ln\frac{R-R_\infty}{R_0-R_\infty}}{k_R}$$

Equations (1) and (2) come from the teachings associated to milk gels, but can be used also to other gels that can develop firmness and that suffers any optical change which allows generating at least an optical parameter that would follow a first order kinetics.

The method of the first aspect of the invention comprises obtaining the value for $k_{GR}$:

- by a calibration process which comprises performing a plurality of optical measures on different gel samples, under predetermined working conditions, and adjusting the value of $k_{GR}$ from the results of said measurements; and/or
- by mathematically modeling its evolution with respect to working conditions and/or gel composition.

For an embodiment, said curd is for making a cheese, and the method comprises selecting the cutting time therefor according to the rheological variable values obtained.

For other embodiments, the method comprises using the rheological variable values obtained for other purposes, such as for stopping some processes applied to the gels, such as upon detecting the hardness at which the gels are refrigerated, if the gel is yogurt or curd to be eaten as food.

A second aspect of the invention concerns to a system for determining gel firmness values from inline optical measurements as defined according to appended independent claim 11. Contrary to the system of US 6,831,741 B1, in the system of the second aspect of the invention said processing means are configured for calculating the value of the rheological variable by processing the collected light data according to an algorithm implementing the expression obtained according to the method of the first aspect of the invention.

For an embodiment, said light means and said optical detection means form part of a scattered light sensor, where said detected light is said emitted light once scattered by said gel and said collected light data is collected scattered light data, and said optical detection means are configured for performing said optical detection and collection of scattered light data using static light scattering.

For an alternative embodiment, said light means and said optical detection means form part of a fluorescence spectroscopy probe, where said detected light is fluorescent light generated by endogen fluorophores of the gel upon said emitted light impinges thereon, and said collected light data being collected fluorescent light data.

According to an embodiment, said light means comprise a first optical fibre and a light source (such a LED for the scattered light sensor or a xenon flash lamp for the spectroscopy probe), arranged for emitting light into the gel through said first optical fibre, the optical detection means comprising a second optical fibre placed adjacent to the first optical fibre and an optical detector arranged for receiving the light transmitted through the second optical fibre coming from the gel, converting them into electrical signals, corresponding to said collected light data, and delivering said electrical signals to the processing means.

As per an embodiment, the processing means of the system of the second aspect of the invention comprise a memory storing at least one reference value for said rheological variable and are configured for comparing the calculated value for said rheological variable with said reference value and for generating a control signal depending on the result of said comparison, and the system further comprises an apparatus connected to said processing means to receive said control signal in order to be controlled thereby.

Said apparatus is, for an embodiment, a cutter for cutting a curd constituting said gel, for cheese making, at an optimum time indicated by said control signal.

Brief Description of the Drawings

The previous and other advantages and features will be more fully understood from the following detailed description of embodiments, with reference to the attached drawings, which must be considered in an illustrative and non-limiting manner, in which:

Figure 1. Light backscatter profile with first and second derivatives as function of time. (I) latent period, (II) sigmoidal period, (III) asymptotic period (Payne and Castillo, 2007).

Figure 2. Light backscatter profile, and its characteristic first and second derivative versus time: $t_{max}$, maximum of the first derivative; $t_{2max}$, maximum of the second derivative; $t_{2min}$, minimum of the second derivative; $t_{max2}$ and $t_{2max2}$ the times to the second maximum of the first and second derivatives; $R_{max}$ and $R_{max2}$, the values of $R$ at $t_{max}$ and $t_{max2}$, respectively (Arango et al., 2013).

Figure 3. Schematic of the coagulation measurement apparatus used to measure near infrared light backscatter during milk coagulation (Tabayehnejad et al., 2011).

Figure 4. Predicted vs. measured storage moduli during milk coagulation at 30°C with 10% solids content. The average value of the experimental data (grey line) at the test conditions (n = 3).

Figure 5. Predicted vs. measured storage moduli during milk coagulation at 30°C with 15% solids content. The average value of the experimental data (grey line) at the test conditions (n = 3).

Detailed Description of Several Embodiments

A complete randomized factorial design with 3 replicates was used to evaluate the proposed expression for optical prediction of G' values (curd firming) using expression (3) during milk coagulation. Milk coagulation tests were conducted with two solids concentration (10 and 15%) and two temperatures (30 and 40 °C) and the coagulation process was simultaneously monitored using an inline light backscatter sensor operated at 880 nm and a small amplitude oscillatory rheometry (SAOR). The whole experiment was run in triplicate. The changes occurred in the protein structure during milk coagulation were correlated to the signal changes derived from the optical sensor and the light backscatter ratio increased as aggregation and gel assembly proceeded.

Skimmed milk powder was reconstituted with distilled water to prepare the milk for the tests. Skimmed milk was selected for the study because it had a constant composition and changes of the composition were unable to affect the results for the study, which allowed minimizing the experimental sources of variation. According to the experimental design two different mixtures of milk were prepared during the study. The milk powder contained ~34% protein. Based on this, the protein content of the mixtures resulted in 3.6 and 5.4% protein for samples containing 10 and 15% solids, respectively. A solution of 525 g anhydrous calcium chloride per litre was used ($CaCl2 \cdot 2H2O$; Panreac Química S.A., Montcada i Reixac, Barcelona, Spain) to enhance milk coagulation. An amount of 0.3 mL of the calcium chloride solution was added per litre of prepared milk. This volume was calculated to deliver 0.157 mg of anhydrous calcium chloride per litre milk. The enzyme used during the experiments was 100% chymosin (CHY-MAX extra; EC 3.4.23.4, isozyme B, 600 IMCU mL-1) supplied by Chr Hansen Inc. (Chr Hansen, Barcelona, Spain). To induce milk coagulation 100 $\mu$L enzyme per litre milk was added.

The milk powder was weighed and afterwards diluted with demineralized water up to 500 mL. This mixture was placed on a stirring plate for 30 min for complete reconstitution of the milk powder in water. After stirring the milk, it was placed for 30 min in a dark place for complete re-hydration of the casein micelles. After this 30 min of rest, calcium chloride was added and the milk was ready for coagulation. The pH of the reconstituted milk was determined and afterwards the milk was put in the water bath to get on temperature. Once the milk reached the target temperature, the enzyme was added and the mixture was stirred thoroughly with a spatula during 20 s. Immediately, two aliquots of -80 mL were placed in the optical sensor vessels and the near infrared light backscatter monitoring software was started. Another aliquot of -40 mL was placed in the rheometer and the respective data acquisition software was launched.

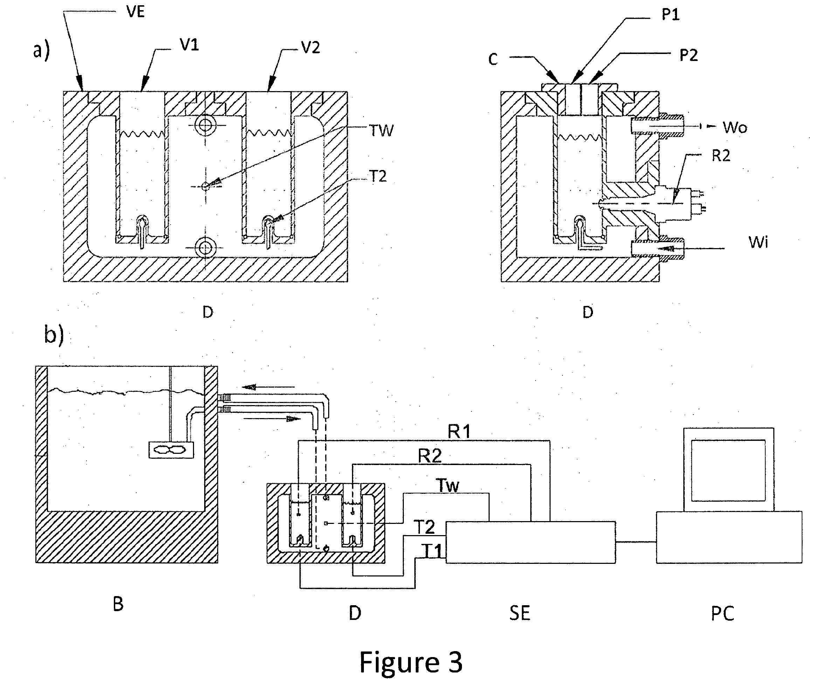

The equipment used to determine the near infrared light backscatter profile at 880 nm during milk coagulation was designed at the University of Kentucky (Fig. 3). A detailed description of it was presented in the work of Tabayehnejad et al. (2011). As shown in Fig. 3a (side view, left, and end view, right), the device D has two vats V1, V2 of 98 mL (Fig. 3a) capacity to monitor coagulation in two samples simultaneously and make accurate comparisons. The device D has a water tank surrounding the vats V1, V2 with a water thermistor Tw placed therein, a water input Wi and a water output Wo for the water tank, vats thermistors T1, T2, optical fibres pairs R1, R2, and an upper cap C, for each vat V1, V2, with a pH port P1 and a stir port P2.

Fig. 3b shows the setup of the total laboratory measurement, where an electronic system SE, which is controlled by a computer PC, has several inputs connected to, respectively, water thermistor Tw, vats thermistors T1, T2, optical fibres pairs R1, R2 for emitting light and receiving the scattered light to be detected by corresponding light detectors (not shown). The circulating water between the water bath B and the outside of the coagulation vats V1, V2, via Wi and Wo, regulated the temperature of milk samples in the vats V1, V2.

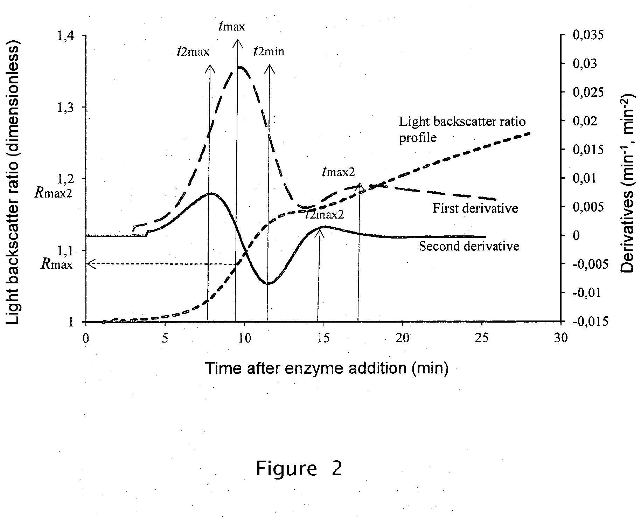

This sensor transmitted near infrared light at 880 nm through two 600 $\mu$m diameter fibres R1, R2. One fibre transmitted infrared radiation into the milk sample while the other fibre transmitted the radiation scattered by the milk particles to a silicon photo-detector. For calibration, the optic sensor was zeroed by excluding light and adjusting the output voltage to 1 V. Response data were collected every 6 s. The initial voltage response ($V_0$) was calculated by averaging the first ten data points after correction for 1 V offset. The light backscatter profile (Fig. 2) was calculated by dividing the voltage output from the detector by the average of the first ten voltage data points collected after the enzyme addition, according to the procedure described by Castillo et al. (2000).

The milk coagulation process was monitored through small amplitude oscillatory rheology (SAOR) by using a ThermoHaake rheometer RS1 (Thermo-Haake GmbH, Karlsruhe, Germany) equipped with a concentric-cylinders sensor (Z34). The tests were done by applying a deformation of 3%, which is within the region of linear viscoelasticity for rennet milk gels (Zoon et al. 1988) and a frequency of 1 Hz. One aliquot of 40 mL of milk with enzyme, which was tempered at the temperature of the test, was deposited in the cylinder of the rheometer, and a thin mineral oil layer was added to the surface of the sample to avoid losses by evaporation and surface cooling. The parameters identified were the elastic module or storage module (G'), the viscous module or loss module (G") and $\tan\delta$ ($\tan\delta = G''/G'$). Gelation time ($t_{G'1}$) was defined as the time when the gels had a G' = 1 Pa.

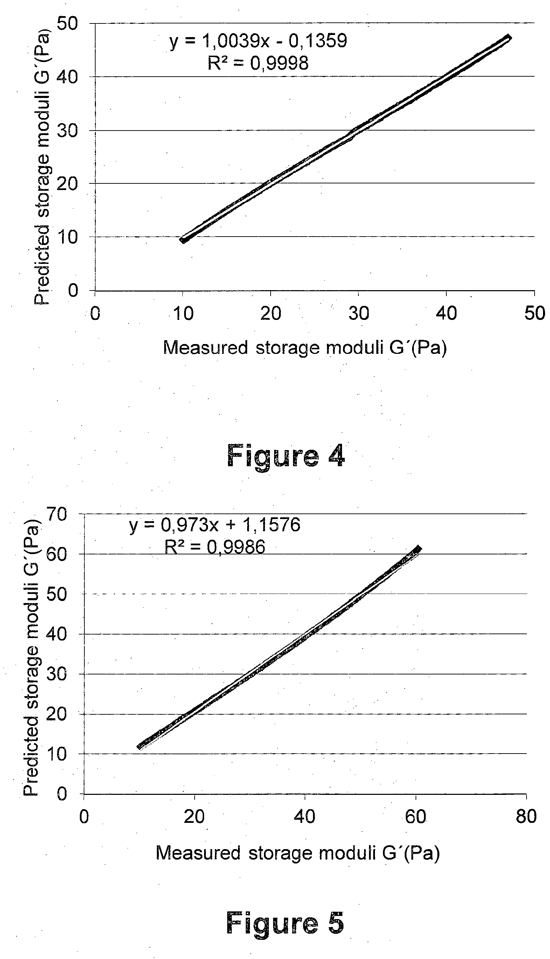

Using the light backscatter ratio obtained with the optic sensor it was possible to predict the curd firming using the proposed expression (3). Figures 4 and 5 show that the fitting was very good in the different coagulation conditions evaluated, where y = predicted G' values, x = observed G' values, and $R^2$ is the determination coefficient for the linear regression between observed and predicted G' values.

The present invention has potential for in-vat control of curd firming during milk coagulation in cheese making, further it can be used for cutting the curd with a specific level of firmness.

Although in the present section only results of experiments using light backscattering have been provided, very similar results can be obtained by applying the above expression (3) to data obtained from other kind of inline optical measurements, particularly from fluorescence light measurements.

A person skilled in the art could introduce changes and modifications in the embodiments described without departing from the scope of the invention as it is defined in the attached claims.

## Claims

1. A method for determining gel firmness values from inline optical measurements, comprising:

   - emitting light into a gel which is hardening to develop firmness;
   - optically detecting light coming from said gel, generated from said emitted light once received therein, to collect light data; and
   - calculating at least one value of a rheological variable from the collected light data and using said at least one calculated value of a rheological variable to determine gel firmness values;

   wherein the method further comprises:

   - defining a mathematical model, valid for the kinetics of the gelation of said gel, of the evolution in time of said rheological variable;
   - defining a mathematical model, valid for the kinetics of the gelation of said gel, of the evolution in time of an optical variable generated from a light generation mechanism responsible for the generation of said detected light;
   - combining both mathematical models in order to obtain an expression which does not include time and which relates said rheological variable with said optical variable; and
   - introducing said collected light data into said expression to calculate said at least one value of the rheological variable,

   wherein:

   - said rheological variable is the storage modulus G';
   - said mathematical model of said optical variable follows the next equation:

   $$R - Ro = (R_\infty - R_0)(1 - e^{-k_R t}) \qquad (1)$$

   where,

   $R - Ro$ = the increase in the light scatter or endogenous fluorescence ratio, after $t_{2min}$, resulting exclusively from gel firming reactions, where $t_{2min}$ is the time at which there is a minimum in the second derivative of a sigmoidal shaped portion of the light scattering or endogenous fluorescence ratio profile corresponding to said evolution in time of said optical variable;
   $R_\infty$ = asymptotic value at infinite time for the light scattering or endogenous fluorescence ratio;
   $k_R$ = a first order rate constant for gel firming reaction;
   $t$ = time after $t_{2min}$;

   - said mathematical model of said rheological variable follows the next equation:

   $$G' = G'_\infty + (G'_0 - G'_\infty)e^{-k_{G'} t} \qquad (2)$$

   where,

   $G'$ = the storage modulus at t time;
   $G'_\infty$ = the value of the storage modulus at t = $\infty$;

$k_{G'}$ = the rate constant for the process; and

- the method comprises solving for time in equation (1) and replacing it into the equation (2), to obtain, as said expression which does not include time and which relates said rheological variable with said optical variable, the next expression:

$$G' = G'_\infty + (G'_0 - G'_\infty) \left(\frac{R-R_\infty}{R_0-R_\infty}\right)^{k_{GR}} \qquad (3)$$

where $k_{GR} = k_{G'}/k_R$ is a constant representing the ratio between the rates of increase of $G'$ and $R$ values as a result of gel firming, and

wherein the value for $k_{GR}$ is obtained by:

- a calibration process which comprises performing a plurality of optical measurements on different gel samples, under predetermined working conditions, and deriving the value of $k_{GR}$ from the results of said calibration and/or
- mathematically modeling the variation of $k_{GR}$ with respect to working conditions and/or gel composition.

2. The method of claim 1, wherein said gel is increasing its turbidity during its hardening, said light generation mechanism is light scattering, said detected light being said emitted light once scattered by said gel and said collected light data being collected scattered light data, and wherein the method comprises using static light scattering for performing said optical detection and collection of scattered light data.

3. The method of claim 1, wherein said gel is increasing its endogenous fluorescence during its hardening, said light generation mechanism is light fluorescence performed by endogen fluorophores of said gel upon said emitted light impinges thereon, said detected light being fluorescent light generated by said endogen fluorophores and said collected light data being collected fluorescent light data.

4. The method of claim 1, 2 or 3, comprising performing said collecting of light data a plurality of times through the hardening and turbidity or endogenous fluorescence increasing of the gel, and calculating the values of the rheological variable for each of said plurality of times.

5. The method of claim 1, 2 or 3, wherein said gel is a casein gel.

6. The method of claim 5, wherein said casein gel is a milk gel.

7. The method of claim 6, wherein said milk gel is a curd, yogurt, fermented/acidified milk or milk derived product.

8. The method of any of the previous claims, wherein said rheological variable is one of the storage modulus $G'$, the viscous modulus $G''$, the phase angle $\delta$, the tangent of said $\delta$ value, tan$\delta$, and a combination thereof.

9. The method of claim 1 or 2 or of any of claims 4 to 8 except when depending on claim 3, wherein said light scattering of both, said optical variable and said optical detection, is at least one of light backscattering and light side scattering.

10. The method of any of the previous claims when depending on claim 7, wherein said curd is for making a cheese, and wherein the method comprises selecting the cutting time therefore according to the rheological variable values obtained.

11. A system for determining gel firmness values from inline optical measurements, comprising:

- light means for emitting light into a gel which is hardening to develop firmness;
- optical detection means for optically detecting light coming from said gel, generated from said emitted light once received therein, to collect light data; and
- processing means for processing the collected light data for calculating the value of a rheological variable to be used to determine gel firmness values;

wherein said processing means are configured for calculating the value of the rheological variable by processing the collected light data according to an algorithm implementing the expression obtained according to the method of claim 1.

12. The system of claim 11, wherein said light means comprise a first optical fibre and a light source arranged for emitting light into said gel through said first optical fibre, said optical detection means comprises a second optical fibre placed adjacent to said first optical fibre and an optical detector arranged for receiving the light transmitted through said second optical fibre coming from the gel, converting them into electrical signals, corresponding to said collected light data, and delivering said electrical signals to the processing means.

13. The system of claim 11 or 12, wherein said processing means comprise a memory storing at least one reference value for said rheological variable and are configured for comparing the calculated value for said rheological variable with said reference value and for generating a control signal depending on the result of said comparison, and wherein the system further comprises an apparatus connected to said processing means to receive said control signal in order to be controlled thereby.

**Patentansprüche**

1. Verfahren zur Bestimmung von Gelfestigkeitswerten anhand optischer Inline-Messungen, umfassend:

   - das Emittieren von Licht in ein aushärtendes Gel, um Festigkeit zu entwickeln;
   - das optische Detektieren von aus dem genannten Gel kommenden Licht, welches aus dem genannten emittierten Licht erzeugt wird, wenn es darin empfängt wird, um Lichtdaten zu sammeln; und
   - das Berechnen von mindestens einem Wert einer rheologischen Variable aus den gesammelten Lichtdaten und das Verwenden von dem genannten mindestens einen berechneten Wert einer rheologischen Variable zur Bestimmung von Gelfestigkeitswerten;

   wobei das Verfahren zusätzlich Folgendes umfasst:

   - das Definieren von einem mathematischen Modell, welches für die Kinetik der Gelierung des genannten Gels, des zeitlichen Verlaufs der genannten rheologischen Variable, gültig ist;
   - das Definieren von einem mathematischen Modell, welches für die Kinetik der Gelierung des genannten Gels, des zeitlichen Verlaufs einer optischen Variable, welche von einem Lichterzeugungsmechanismus erzeugt wird, welcher für die Erzeugung des genannten detektierten Lichts zuständig ist, gültig ist;
   - das Kombinieren von beiden mathematischen Modellen, um eine Ausdruck zu erhalten, welcher keine Zeit beinhaltet und welcher die genannte rheologische Variable zu der genannten optischen Variable in Beziehung setzt; und
   - das Einführen von den genannten gesammelten Lichtdaten in den genannten Ausdruck, um den genannten mindestens einen Wert der rheologischen Variable zu berechnen,

   wobei:

   - die genannte rheologische Variable der Speichermodul $G'$ ist;
   - das genannte mathematische Modell der genannten optischen Variable die folgende Gleichung folgt:

$$R - Ro = (R_\infty - R_0)(1 - e^{-k_R t}) \qquad (1)$$

   in welcher,

   $R - Ro$ = die Erhöhung bei der Lichtstreuung oder dem endogenen Fluoreszenzverhältnis, nach $t_{2min}$, welche sich ausschließlich aus den Gelfestigkeitsreaktionen ergibt, wobei $t_{2min}$ die Zeit ist, in welcher es ein Minimum in der zweiten Ableitung eines sigmoidalförmigen Teils der Lichtstreuung oder des endogenen Fluoreszenzverhältnisprofils gibt, welches dem genannten zeitlichen Verlauf der genannten optischen Variable entspricht;
   $R_\infty$ = asymptotischer Wert zu einer unendlichen Zeit für die Lichtstreuung oder das endogene Fluoreszenzverhältnis;
   $k_R$ = eine Geschwindigkeitskonstante erster Ordnung für eine Gelfestigkeitsreaktion;

$t$ = Zeit nach t$_{2min}$;

- das genannte mathematische Modell der genannten rheologischen Variable die folgende Gleichung folgt:

$$G' = G'_\infty + (G'_0 - G'_\infty)e^{-k_{G'}t} \qquad (2)$$

in welcher,

$G'$ = der Speichermodul bei der Zeit t;
$G'_\infty$ = der Wert des Speichermoduls bei t =$\infty$;
$k_{G'}$ = die Geschwindigkeitskonstante für den Vorgang; und

- das Verfahren das Auflösen der Gleichung (1) nach der Zeit und das Ersetzen derselben in Gleichung (2) umfasst, um den folgenden Ausdruck, als der genannte Ausdruck, welcher keine Zeit beinhaltet und welcher die genannte rheologische Variable zu der genannten optischen Variable in Beziehung setzt, zu erhalten:

$$G' = G'_\infty + (G'_0 - G'_\infty)\left(\frac{R-R_\infty}{R_0-R_\infty}\right)^{k_{GR}} \qquad (3)$$

in welcher $k_{GR}$ = $k_{G'}/k_R$ eine Konstante ist, welche das Verhältnis zwischen den Erhöhungsgeschwindigkeiten der $G'$ und $R$-Werte als Ergebnis der Gelfestigkeit darstellt, und

wobei der Wert für $k_{GR}$ aus dem Folgenden erhalten wird:

- einem Kalibrierungsvorgang, welcher das Durchführen einer Vielzahl von optischen Messungen bei unterschiedlichen Gelproben, unter vorbestimmten Arbeitsbedingungen, und das Ableiten von dem Wert von $k_{GR}$ aus den Ergebnissen der genannten Kalibrierung umfasst, und/oder
- dem mathematischen Modellieren der Variation von k$_{GR}$ in Bezug auf die Arbeitsbedingungen und/oder die Gelzusammensetzung.

2. Verfahren nach Anspruch 1, wobei sich die Trübheit des genannten Gels während des Aushärtens desselben erhöht, der genannte Lichterzeugungsmechanismus Lichtstreuung ist, das genannte detektierte Licht das genannte emittierte Licht ist, wenn es vom genannten Gel gestreut wird ist und die genannten gesammelten Lichtdaten gesammelte gestreute Lichtdaten sind, und wobei das Verfahren das Verwenden von statischer Lichtstreuung für das Durchführen der genannten optischen Detektion und Sammlung von gestreuten Lichtdaten umfasst.

3. Verfahren nach Anspruch 1, wobei sich die endogene Fluoreszenz des genannten Gels während der Aushärtung desselben erhöht, der genannte Lichterzeugungsmechanismus Lichtfluoreszenz ist, welche von endogenen Fluorophoren des genannten Gels nachdem das genannte emittierte Licht darauf auftrifft durchgeführt wird, wobei das genannte detektierte Licht Fluoreszenzlicht ist, welches von den genannten endogenen Fluorophoren erzeugt wird und die genannten gesammelten Lichtdaten gesammelte Fluoreszenzlichtdaten sind.

4. Verfahren nach Anspruch 1, 2 oder 3, umfassend das mehrmalige Durchführen der genannten Sammlung von Lichtdaten während der Aushärtungs- und Trübheits- oder endogenen Fluoreszenzerhöhung des Gels, und das Berechnen von den Werten der rheologischen Variable für jede der mehrmaligen Durchführungen.

5. Verfahren nach Anspruch 1, 2 oder 3, wobei das genannte Gel ein Caseingel ist.

6. Verfahren nach Anspruch 5, wobei das genannte Caseingel ein Milchgel ist.

7. Verfahren nach Anspruch 6, wobei das genannte Milchgel Quark, Joghurt, fermentierte/gesäuerte Milch oder ein aus Milch gewonnenes Produkt ist.

8. Verfahren nach einem der vorhergehenden Ansprüche, wobei die genannte rheologische Variable eines aus dem Speichermodul $G'$, dem Viskositätsmodul $G''$, dem Phasenwinkel $\delta$, der Tangente des genannten $\delta$-Werts, tan$\delta$,

und einer Kombination derselben ist.

9. Verfahren nach Anspruch 1 oder 2 oder nach einem der Ansprüche 4 bis 8 außer wenn sie von Anspruch 3 abhängig sind, wobei die genannte Lichtstreuung von beiden, der genannten optischen Variable und der genannten optischen Detektion, mindestens eine aus Lichtrückstreuung und seitlicher Lichtstreuung ist.

10. Verfahren nach einem der vorhergehenden Ansprüche wenn sie von Anspruch 7 abhängig sind, wobei der genannte Quark zur Herstellung eines Käses ist, und wobei das Verfahren daher das Auswählen der Bearbeitungszeit gemäß den erhaltenen Werten der rheologischen Variable umfasst.

11. System zur Bestimmung von Gelfestigkeitswerten anhand optischer Inline-Messungen, umfassend:

   - Lichtmittel zum Emittieren von Licht in ein aushärtendes Gel, um Festigkeit zu entwickeln;
   - optische Detektionsmittel zum optischen Detektieren von aus dem genannten Gel kommenden Licht, welches aus dem emittierten Licht erzeugt wird, wenn es darin empfängt wird, um Lichtdaten zu sammeln; und
   - Verarbeitungsmittel für die Verarbeitung der gesammelten Lichtdaten zum Berechnen des Werts einer rheologischen Variable, welche zur Bestimmung von Gelfestigkeitswerten verwendet werden soll;

   wobei die genannten Verarbeitungsmittel dazu ausgebildet sind, den Wert der rheologischen Variable mittels der Verarbeitung der gesammelten Lichtdaten gemäß einem Algorithmus zu berechnen, welcher den gemäß dem Verfahren nach Anspruch 1 erhaltenen Ausdruck implementiert.

12. System nach Anspruch 11, wobei die genannten Lichtmittel eine erste optische Faser und eine Lichtquelle umfassen, welche dazu angeordnet ist, Licht in das genannte Gel durch die genannte erste optische Faser zu emittieren, wobei die genannten optischen Detektionsmittel eine zweite optische Faser, welche der ersten optischen Faser benachbart platziert ist, und einen optischen Detektor, welcher dazu angeordnet ist, das durch die zweite optische Faser übertragene, aus dem Gel kommende Licht zu empfangen, umfasst, so dass sie in elektrische Signale umwandelt werden, welche den genannten gesammelten Lichtdaten entsprechen, und die genannten elektrischen Signale den Verarbeitungsmitteln zugeführt werden.

13. System nach Anspruch 11 oder 12, wobei die genannten Verarbeitungsmittel einen Speicher umfassen, welcher mindestens einen Referenzwert für die genannte rheologische Variable speichert, und dazu ausgebildet sind, den berechneten Wert für die genannte rheologische Variable mit dem genannten Referenzwert zu vergleichen und ein Steuersignal in Abhängigkeit des Ergebnisses des genannten Vergleichs zu erzeugen, und wobei das System zusätzlich eine mit den genannten Verarbeitungsmitteln verbundene Einrichtung umfasst, um das genannte Steuersignal zu empfangen, so dass sie dadurch gesteuert wird.

**Revendications**

1. Une méthode pour déterminer les valeurs de fermeté d'un gel des mesures optiques en ligne, comportant :

   - l'émission de lumière à un gel qui est en train de durcir pour développer une fermeté ;
   - la détection optique de la lumière provenant de ce gel, générée à partir de cette lumière émise lorsqu'elle y a été reçue, pour collecter des données de lumière ; et
   - le calcul d'au moins une valeur d'une variable rhéologique à partir des données de lumière collectées et l'utilisation de cette au moins une valeur calculée d'une variable rhéologique pour déterminer les valeurs de fermeté du gel ;

   dans laquelle la méthode comporte en plus :

   - la définition d'un modèle mathématique, valable pour la cinétique de la gélification de ce gel, de l'évolution dans le temps de cette variable rhéologique ;
   - la définition d'un modèle mathématique, valable pour la cinétique de la gélification de ce gel, de l'évolution dans le temps d'une variable optique générée à partir d'un mécanisme de génération de lumière responsable de la génération de cette lumière détectée ;
   - la combinaison des deux modèles mathématiques afin d'obtenir une expression qui ne comprenne pas le temps et qui relie cette variable rhéologique à cette variable optique ; et

- l'introduction de ces données de lumière collectées dans cette expression pour calculer cette au moins une valeur de la variable rhéologique ;

dans laquelle :

- cette variable rhéologique est le module de stockage G' ;
- ce modèle mathématique de cette variable optique suit l'équation suivante :

$$R - Ro = (R_\infty - R_0)\ (1 - e^{-k_R t}) \tag{1}$$

où,

$R - R_0$ = l'augmentation du rapport de dispersion de lumière ou de fluorescence endogène après $t_{2min}$, résultant exclusivement des réactions de raffermissement du gel, où $t_{2min}$ est le temps dans lequel il y a un minimum dans la deuxième dérivée d'une portion en forme sigmoïdale du profil de rapport de dispersion de lumière ou de fluorescence endogène correspondant à cette évolution dans le temps de cette variable optique ;
$R_\infty$ = valeur asymptotique à temps infini pour le rapport de dispersion de lumière ou de fluorescence endogène ;
$k_R$ = une constante de vitesse de premier ordre pour la réaction de raffermissement du gel ;
$t$ = temps après $t_{2min}$ ;

- ce modèle mathématique de cette variable rhéologique suit l'équation suivante :

$$G' = G'_\infty + (G'_0 - G'_\infty)e^{-kG't} \tag{2}$$

où,

$G'$ = le module de stockage au temps t ;
$G'_\infty$ = la valeur du module de stockage à t = ∞ ;
$k_{G'}$ = la constante de vitesse pour le processus ; et

- la méthode comprend résoudre pour le temps dans l'équation (1) et la remplacer par l'équation (2), pour obtenir, comme cette expression qui ne comprend pas le temps et qui relie cette variable rhéologique à cette variable optique, l'expression suivante :

$$G' = G'_\infty + (G'_0 - G'_\infty)\left(\frac{R - R_\infty}{R_0 - R_\infty}\right)^{kGR} \tag{3}$$

où $k_{GR} = k_{G'}/k_R$ est une constante représentant le rapport entre les vitesses d'augmentation des valeurs de $G'$ et $R$ comme résultat de l'affermissement du gel, et

dans laquelle la valeur pour $k_{GR}$ est obtenue par :

- un processus d'étalonnage qui comprend effectuer une pluralité de mesures optiques sur différents échantillons de gel, sous des conditions de travail prédéterminées et dériver la valeur de $k_{GR}$ des résultats de cet étalonnage et/ou
- la modélisation mathématique de la variation de $k_{GR}$ par rapport aux conditions de travail et/ou composition du gel.

2. La méthode de la revendication 1, dans laquelle ce gel augmente sa turbidité durant son durcissement, ce mécanisme de génération de lumière est disperseur de lumière, cette lumière détectée étant cette lumière émise lorsqu'elle est dispersée par ce gel et ces données de lumière collectées étant des données de lumière dispersée collectées et dans laquelle la méthode comporte l'utilisation de dispersion de lumière statique pour effectuer cette détection optique et collecter des données de lumière diffusée.

3. La méthode de la revendication 1, dans laquelle ce gel augmente sa fluorescence endogène durant son durcissement, ce mécanisme de génération de lumière est la fluorescence de lumière effectuée par des fluorophores endogènes de ce gel lorsque cette lumière émise le touche, cette lumière détectée étant une lumière fluorescente générée par ces fluorophores endogènes et ces données de lumière collectées étant des données de lumière fluorescente collectées.

4. La méthode de la revendication 1, 2 ou 3, comportant effectuer cette collecte de données de lumière une pluralité de temps par le biais du durcissement et la turbidité ou de l'augmentation de la fluorescence endogène du gel et calculer les valeurs de la variable rhéologique pour chacune de cette pluralité de temps.

5. La méthode de la revendication 1, 2 ou 3, dans laquelle ce gel est un gel de caséine.

6. La méthode de la revendication 5, dans laquelle ce gel de caséine est un gel de lait.

7. La méthode de la revendication 6, dans laquelle ce gel de lait est du lait caillé, yaourt, lait fermenté/acidifié ou un produit dérivé du lait.

8. La méthode d'une quelconque des revendications précédentes, dans laquelle cette variable rhéologique est un des modules de stockage G', le module visqueux G", l'angle de phase $\delta$, la tangente de cette valeur $\delta$, tan$\delta$, et une de leurs combinaisons.

9. La méthode de la revendication 1 ou 2 ou d'une quelconque des revendications 4 à 8, sauf lorsqu'elle dépend de la revendication 3, dans laquelle cette dispersion de lumière des deux, cette variable optique et cette détection optique, est au moins une de rétrodispersion de lumière et de dispersion latérale de lumière.

10. La méthode d'une quelconque des revendications précédentes lorsqu'elle dépend de la revendication 7, dans laquelle ce lait caillé est pour fabriquer un fromage et dans laquelle cette méthode comporte la sélection du temps de coupe donc conformément aux valeurs de variable rhéologique obtenues.

11. Un système pour déterminer les valeurs de fermeté du gel à partir de mesures optiques en ligne, comportant :

- des moyens de lumière pour émettre de la lumière à un gel qui est en train de durcir pour développer sa fermeté ;
- des moyens de détection optique pour la détection optique de la lumière en provenance de ce gel générée à partir de cette lumière émise dès qu'elle y est reçue, pour collecter des données de lumière ; et
- des moyens de traitement pour traiter les données de lumière collectées pour calculer la valeur d'une variable rhéologique à utiliser pour déterminer les valeurs de fermeté du gel ;

dans laquelle ces moyens de traitement sont configurés pour calculer la valeur de la variable rhéologique par le traitement de données de lumière collectées conformément à un algorithme mettant en oeuvre l'expression obtenue conformément à la méthode de la revendication 1.

12. Le système de la revendication 11, dans lequel ces moyens de lumière comportent une première fibre optique et une source de lumière aménagée pour émettre de la lumière à ce gel à travers cette première fibre optique, ces moyens de détection optique comportent une deuxième fibre optique placée adjacente à cette première fibre optique et un détecteur optique aménagé pour recevoir la lumière transmise à travers cette deuxième fibre optique provenant du gel, en les convertissant en signaux électriques correspondant à ces données de lumière collectées et délivrer ces signaux électriques aux moyens de traitement.

13. Le système de la revendication 11 ou 12, dans lequel ces moyens de traitement comportent une mémoire qui stocke au moins une valeur de référence pour cette variable rhéologique et sont configurés pour comparer la valeur calculée pour cette variable rhéologique avec cette valeur de référence et pour générer un signal de contrôle dépendant du résultat de cette comparaison et dans lequel le système comporte en plus un appareil relié à ces moyens de traitement pour recevoir ce signal de contrôle afin d'être ainsi contrôlé.

Figure 1

Figure 2

Figure 3

$y = 1,0039x - 0,1359$
$R^2 = 0,9998$

Figure 4

$y = 0,973x + 1,1576$
$R^2 = 0,9986$

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6831741 B1

**Non-patent literature cited in the description**

- **ARANGO, O. ; TRUJILLO, A. J. ; CASTILLO, M.** Influence of fat replacement by inulin on rheological properties, kinetics of rennet milk coagulation, and syneresis of milk gels. *Journal of Dairy,* 2013, vol. 96, 1984-1996
- **BENGUIGUI, L. ; EMERY, J. ; DURAND, D. ; BUSNEL, J. P.** Ultrasonic study of milk-clotting. *Lait,* 1994, vol. 74, 197-206
- **COSGROVE, N.** Design and development of an ultrasonic measuring system to monitor milk coagulation. National University of Ireland for award of MEngnSc, 2000
- **CARLSON, A. ; HILL JR., C.G. ; OLSON, N.E.** Kinetics of milk coagulation. III. Mathematical modelling of the kinetics of curd formation following enzymatic hydrolysis of K-casein- parameter estimation. *Biotechnol. Bioeng.,* 1987, vol. 29, 601-611
- Cutting time prediction methods in cheese making. **CASTILLO, M.** Encyclopedia of Agricultural, Food, and Biological Engineering. Taylor & Francis Group, 2006, vol. 1, 1-7
- **CASTILLO, M. ; PAYNE, F.A. ; HICKS, C.L. ; LAENCINA, J.S. ; LOPEZ, M.B.** Modeling casein aggregation and curd firming in goats' milk from backscatter of infrared light. *J. Dairy Res.,* 2003, vol. 70, 335-348
- **CASTILLO, M. ; PAYNE, F. A. ; HICKS, C. L. ; LAENCINA, J. ; LOPEZ, M. B.** Effect of calcium and enzyme in cutting time prediction of coagulating goats' milk using a light scattering sensor. *International Dairy Journal,* 2002, vol. 12, 1019-1023
- **CASTILLO, M. ; PAYNE, F. A. ; HICKS, C. L. ; LOPEZ, M. B.** Predicting cutting and clotting time of coagulating goats' milk using diffuse reflectance: Effect of pH, temperature and enzyme concentration. *International Dairy Journal,* 2000, vol. 10, 551-562
- Cutting time prediction during cheese making by near infrared light backscattering. **CASTILLO, M.** PhD thesis. University of Murcia, 2001
- The formation and properties of biopolymer gels. **CLARK, A.H. ; AMICI, E.H.** Food Colloids and Materials. Royal Society of Chemistry, 2003, 35-48

- **DALGLEISH, D. G. ; VERESPEJ, E. ; ALEXANDER, M. ; CORREDIG, M.** The ultrasonic properties of skim milk related to the release of calcium from casein micelles during acidification. *International Dairy Journal,* 2005, vol. 15, 1105-1112
- **DEJMEK, P.** Dynamic rheology of rennet curd. *J. Dairy Sci.,* 1987, vol. 70, 1325-1330
- **DOUILLARD, R.** Rheological analysis of curd formation. *J. Texture Studies,* 1973, vol. 4, 158-165
- **FOX, P. F. ; MCSWEENEY, P. L. ; COGAN, T. M. ; GUINEE, T. P.** Cheese: Chemistry, Physics and Microbiology. An Aspen Publication, 2004, 404
- **HARDY, J. ; FANNI, J.** Application of reflection photometry to the measurement of milk coagulation. *J. Food Sci.,* 1981, vol. 46, 1956-1957
- **HEMAR, Y. ; SINGH, H. ; HOME, D. S.** Determination of early stages of rennet-induced aggregation of casein micelles by diffusing wave spectroscopy and rheological measurements. *Current Applied Physics,* 2004, vol. 4, 362-365
- **HORI, T.** Objective measurements of the process of curd formation during rennet treatment of milks by the hot-wire method. *Journal of Food Science,* 1985, vol. 50, 911-917
- **JOHNSTON, K.A. ; LUCKMAN, M.S. ; LILLEY, H.G. ; SMALE, B.M.** Effect of various cutting and stirring conditions on curd particle size and losses of fat to the whey during cheddar cheese manufacture in Ost vats. *Int. Dairy J.,* 1998, vol. 8, 281-288
- **KLANDAR, A.H. ; LAGAUDE, A. ; CHEVALIER-LUCIA D.** Assessment of the rennet coagulation of skim milk: A comparison of methods. *International Dairy Journal,* 2007, vol. 17, 1151-1160
- **LAGAUDE A. ; FERNANDEZ L. ; CUQ J.-L. ; MARCHESSEAU S.** Characterization of curd formation during the rennet coagulation of milk by an optical microscopic method. *International Dairy Journal,* 2004, vol. 14, 1033-1039
- **LAPORTE, M.-F. ; MARTEL, R. ; PAQUIN, P.** The near-infrared optic probe for monitoring rennet coagulation in cows' milk. *International Dairy Journal,* 1998, vol. 8, 659-666

- **LUCEY, J. A.** Formation and physical properties of milk protein gels. *Journal of Dairy Science,* 2002, vol. 85, 281-294
- **MCMAHON, D. J. ; BROWN, R. J.** Evaluation of formagraph for comparing rennet solutions. *Journal of Dairy Science,* 1982, vol. 65, 1639-1642
- **MCMAHON, D.J. ; BROWN, R.J. ; RICHARDSON, G.H. ; ERNSTROM, C.A.** Effects of calcium, phosphate, and bulk culture media on milk coagulation properties. *J. Dairy Sci.,* 1984, vol. 67, 930-938
- **MELLEMA, M. ; WALSTRA, P. ; VAN OPHEUSDEN, J. H. J. ; VAN VLIET, T.** Effects of structural rearrangements on the rheology of rennet-induced casein particle gels. *Advances in Colloid and Interface Science,* 2002, vol. 98, 25-50
- **NAJERA, A. I. ; DE RENOBALES, M. ; BARRON, L. J. R.** Effects of pH, temperature, CaCl2 and enzyme concentrations on the rennet-clotting properties of milk: A multifactorial study. *Food Chemistry,* 2003, vol. 80, 345-352
- **NICOLAU, N. ; CASTILLO, M. ; BUFFA, M ; O'CALLAGHAN ; D. J., GUAMIS, B.** Estudio de la coagulacion de leche de oveja mediante monitorización con un sensor óptico. *Actas del VI Congreso Español de Ingeniería de Alimentos,* 2010
- **O'CALLAGHAN, D. J. ; O'DONNELL, C. P. ; PAYNE, F. A.** Review of systems for monitoring curd setting during cheesemaking. *International Journal of Dairy Technology,* 2002, vol. 55, 65-74
- **O'CALLAGHAN, D. J. ; MULHOLLAND, E. P. ; DUFFY, A. P. ; O'DONNELL, C. P. ; PAYNE, F. A.** Evaluation of hot wire and optical sensors for online monitoring of curd firmness during milk coagulation. *Irish Journal of Agricultural and Food Research,* 2001, vol. 40, 227-238
- **O'CALLAGHAN, D. J. ; O'DONNELL, C. P. ; PAYNE, F. A.** On-line sensing techniques for coagulum setting in renneted milks. *Journal of Food Engineering,* 2000, vol. 43, 155-165
- **O'CALLAGHAN, D. J. ; O'DONNELL, C. P. ; PAYNE, F. A.** A comparison of on-line techniques for determination of curd setting time using cheese milks under different rates of coagulation. *Journal of Food Engineering,* 1999, vol. 41, 43-54
- **PASSOS, E. F. ; MONTEIRO, P. S. ; OLIVEIRA, R. C. ; MARTINS, J. G. O. ; ALVES, H. G. ; BRANDAO, S. C. C.** Predicting the cutting time of coagulating milk for cheese production using a heated thermistor. *Journal of Food Science,* 1999, vol. 64, 879-882
- Light backscatter sensor applications in milk coagulation. **PAYNE, F. A. ; CASTILLO M.** Encyclopedia of Agricultural, Food, and Biological Engineering. Taylor & FrancisGroup, 2007, vol. 1, 1-5
- **PAYNE, F.A. ; HICKS, C.L. ; SHEN, P.S.** Predicting optimal cutting time of coagulating milk using diffuse reflectance. *J. Dairy Sci.,* 1993, 7648-7661
- Fiber optic milk coagulation sensor for cut-time detection. **PAYNE, F.A. ; MADANGOPAL, S. ; HICKS, C.L. ; SHEARER, S.A.** Food Processing Automation Conference. American Society of Agricultural Engineers, 1990, vol. 02-90, 173
- **PICQUE, D. ; CATTENOZ, T. ; LATRILLE, E. ; PERRET, B. ; CORRIEU, G.** Dispositif et procede de controle de la fabrication de fromages. *INRA,* 1996
- **SAPUTRA, D. ; PAYNE, F.A. ; LODDER, R.A. ; SHEARER, S.A.** Selection of near-infrared wavelengths for monitoring milk coagulation using principal component analysis. Trans. *ASAE,* 1992, vol. 35, 1597-1605
- **SCOTT BLAIR, G.W. ; BURNETT, J.** Physical changes in milk caused by the action of rennet..1. *Dairy Res.,* 1958, vol. 25, 297-303
- **TABAYEHNEJAD, N. ; CASTILLO, M. ; PAYNE, F. A.** Comparison of total milk-clotting activity measurement precision using the Berridge clotting time method and a proposed optical method. *Journal of Food Engineering,* 2011, vol. 108, 549-556
- **TAIFI, N. ; BAKKALI, F. ; FAIZ, B. ; MOUDDEN, A. ; MAZE, G. ; DECULTOT, D.** Characterization of the syneresis and the firmness of the milk gel using an ultrasonic technique. *Measurement Science and Technology,* 2006, vol. 17, 281-287
- **TOKITA, M.K. ; KIKICHI, R. ; NIKI, R. ; ARIMA, S.** Dynamic viscoelastic studies on the mechanism of milk clotting process. *Biorheology,* 1982, vol. 19, 209-219
- **TUSZYNSKI, W.B.** A kinetic model of the clotting of casein by rennet. *J. Dairy Res.,* 1971, vol. 38, 113-125
- **ZHONG, Q. ; DAUBERT, C. R.** Kinetics of rennet casein gelation at different cooling rates. *Journal of Colloid and Interface Science,* 2004, vol. 279, 88-94
- **MANUEL Z CASTILLO.** Modelling casein aggregation and curd firming in goats milk from backscatter of infrared light. *Journal of Dairy Research,* 01 August 2003, vol. 70 (3), 335-348